# EUROPÄISCHE PATENTSCHRIFT

(11) **EP 2 335 745 B1**
(45) Veröffentlichungstag und Bekanntmachung des Hinweises auf die Patenterteilung: **22.11.2017**
(21) Anmeldenummer: 10192871.1
(22) Anmeldetag: 29.11.2010
(51) Int. Cl.: A61L 29/08, A61L 29/16, A61L 31/10, A61L 31/16

(54) **Implantat mit Beschichtung**
Implant with coating
Implant doté d'un revêtement

(30) Priorität: 21.12.2009 US 288347 P
(43) Veröffentlichungstag der Anmeldung: 22.06.2011
(73) Patentinhaber: Biotronik VI Patent AG, 6341 Baar (CH)
(72) Erfinder: Hofmann, Andreas, 91322 Gräfenberg (DE); Borck, Alexander, 91086 Aurachtal (DE); Tittelbach, Michael, 90429 Nürnberg (DE)
(74) Vertreter: Randoll, Sören

(56) Entgegenhaltungen:
- EP-A1- 1 916 006
- EP-A1- 2 243 501
- EP-A2- 1 003 571
- US-A1- 2006 078 588
- US-A1- 2006 286 139
- STEIGERWALD K ET AL: "The pre-clinical assessment of rapamycin-eluting, durable polymer-free stent coating concepts", BIOMATERIALS, ELSEVIER SCIENCE PUBLISHERS BV., BARKING, GB, Bd. 30, Nr. 4, 1. Februar 2009 (2009-02-01), Seiten 632-637, XP025693621, ISSN: 0142-9612, DOI: 10.1016/J.BIOMATERIALS.2008.10.005 [gefunden am 2008-11-05]

## Beschreibung

Die Erfindung betrifft ein medizinisches Implantat mit einer wenigstens eine pharmazeutisch aktive Substanz enthaltenden Beschichtung.

### Stand der Technik und Hintergrund der Erfindung

Implantate, wie Stents oder Katheter sind Röhrchen oder Schläuche verschiedenen Durchmessers, die in den jeweiligen zu behandelnden Körperhohlraum eingeführt werden können. Sogenannte Ballonkatheter, die vor allem in der Angioplastie zur Erweiterung oder Wiedereröffnung eines Gefäßes eingesetzt werden, werden in Kombination mit einem Führungsdraht appliziert, der zuerst in das zu behandelnde Gefäß eingeschoben wird. Anschließend wird ein Schlauch, der in einem vorgegebenen Bereich des Schlauchs einen nicht dilatierten, gefalteten Ballon aufweist, entlang des Führungsdrahts bis zu der zu behandelnden Stelle des Gefäßes vorgeschoben, so dass der Ballon im Bereich der zu behandelnden Stelle des Gefäßes, die z.B. eine Stenose aufweist, platziert ist. Danach wird der Ballon dilatiert, d.h. entfaltet und aufgedehnt, so dass die zu behandelnde Stelle wiederer-öffnet oder erweitert wird und der Strom der Körperflüssigkeit in dem Gefäß nicht oder nicht mehr in dem zuvor vorhandenen Maße behindert wird. Schließlich wird der Ballon wieder entleert und entlang des Führungsdrahts wieder aus dem Gefäß entfernt. Gleichzeitig oder anschließend wird auch der Führungsdraht aus dem Gefäß zurückgezogen.

In den letzten Jahren wurden verschiedene medikamentenbeschichtete Stents und Ballonkatheter entwickelt und für die Behandlung koronarer und peripherer Gefäßerkrankungen am Menschen zugelassen. Die Medikamentenbeschichtung wird auf die Oberfläche des Stents oder Ballonkatheters aufgebracht. Der Katheter wird über einen Führungsdraht und durch einen Führungskatheter bis zur Läsion vorgeschoben. Während dieser Prozedur ist der Katheter verschiedenen mechanischen Einflüssen ausgesetzt, die zum Abrieb und Verlust der Beschichtung führen können. Hierzu zählen unter anderem Abziehen des Protektors, Berühren des Ballons durch den Operateur, Knicken des Ballons, Durchführen des Katheters durch den sogenannten Introducer oder durch eine Schleuse, Reibung im Führungskatheter, Kontakt mit Blut, Reibung an der Gefäßwand, die vor allem bei calzifizierten Läsionen einen hohen mechanischen Widerstand darstellt.

Der Verlust von pharmakologisch aktiver Substanz schon auf den Weg zum Zielort macht eine deutlich höhere Wirkstoffbeladung des Stents oder Ballonkatheters nötig, als therapeutisch tatsächlich notwendig ist.
Bei der Wirkstoffbeschichtung müssen demnach zwei Ziele erreicht werden: den reproduzierbar effektiven Transport des Wirkstoffs bis zum Ort der Anwendung und die rasche Freisetzung einer ausreichenden Dosis im Moment der Stent bzw. Ballondilatation an die Gefäßwand. Allerdings sind die Wechselwirkungen Stent- oder Ballonoberfläche/Wirkstoff limitiert, zusätzlich neigen viele Wirkstoffe dazu, Kristallite oder allgemein Festphasen zu bilden, die brüchig sind und durch mechanische Belastung zur Desintegration neigen. Bisher werden medikamentenbeschichtete Implantate entweder so hergestellt, dass die Beschichtung stabil ist, aber nur geringfügig Substanz an die Läsion abgibt, oder so, dass die Beschichtung mechanisch fragil ist, und einen Wirkstoffverlust vor Applikation am Ort der Läsion aufweist.

Zugelassene, medikamentenbeschichtete Stents oder Ballonkatheter für die Behandlung koronarer und peripherer Gefäßerkrankungen am Menschen basieren insbesondere auf der Verwendung des Wirkstoffes Paclitaxel. Paclitaxel ist ein stark hydrophobes Molekül, das zur Bildung kristalliner Strukturen neigt. Die Kristallinität der Substanz limitiert die Bioverfügbarkeit; das Kristallgitter muss aufgebrochen werden, damit der Wirkstoff solvatisiert und damit verfügbar vorliegt. Ferner werden durch Reibungskräfte des Ballons im Führungskatheter und im Gefäßsystem Teile der Wirkstoffbeschichtung abgelöst, wenn die Beschichtung zu geringe mechanische Stabilität aufweist. Um einen möglichst guten Übergang zwischen Ballon und Gewebe zu gewährleisten, sollte der Wirkstoff in wenig kristalliner Form vorliegen, um die Aufnahme in die Gefäßwand zu erleichtern. Die Bildung geeigneter Morphologien des Wirkstoffs kann durch den Zusatz unterschiedlicher, pharmakologischer Hilfsstoffe (Exzipient) unterstützt werden. Als Exzipient werden beispielsweise Iopromide und Harnstoff verwendet. Obwohl diese Beschichtungen sehr leicht ablösbar sind und ein Großteil der Beschichtung während der Intervention verloren geht, konnte eine gute klinische Wirksamkeit gezeigt werden. Dies macht deutlich, dass eigentlich ein Bruchteil der Wirkstoffbeladung ausreicht, um einen klinisch wünschenswerten Effekt zu erzielen. Andere Hersteller versuchen durch Kavitäten auf der Ballonoberfläche den Abrieb des Wirkstoffs zu verhindern oder durch Abscheidung einer porösen Paclitaxel Phase die Löslichkeit des Wirkstoffs zu erhöhen.

US 2006/0286139 A1 beschreibt Implantate mit Beschichtung. In Biomaterials des Jahres 2009, 30, S. 632-637 werden Wirkstoff-eluierende Stents beschrieben. In EP 1916006 A1 werden beschichtete Implantate offenbart. In EP 2243501 A1 werden beschichtete Ballons beschrieben. Es besteht demnach ein Bedarf nach einer Lösung, die Wirkstoffbeladung des Implantats zu reduzieren, wobei gleichzeitig die Verfügbarkeit von Wirkstoff am Anwendungsort nicht vermindert wird, beim Weg dorthin jedoch deutlich weniger Verluste auftreten. Durch die geringere Dosierung wäre die Gesamtbelastung für den Patienten reduziert und die Reproduzierbarkeit der tatsächlich therapeutisch aktiv werdenden Dosis erhöht.

### Erfindungsgemäße Lösung

Eines oder mehrere der geschilderten Probleme lassen sich mit Hilfe des erfindungsgemäßen Implantats mit einer wenigstens eine pharmazeutisch aktive Substanz enthaltenden Beschichtung lösen oder zumindest mindern. Das erfindungsgemäße Implantat zeichnet sich dadurch aus, dass die Beschichtung von einer Schutzschicht bedeckt ist, die aus einem oder mehreren Materialien der Gruppe umfassend Vinylacetat-Crotonsäure-Copolymer, Vinylacetat-Vinylpropionat-Crotonsäure-Terpolymer, Methylvinylether-Maleinsäureanhydrid-Copolymer, Vinylpyrrolidon-Dimethylaminoethylacrylat-Copolymer, Polyvinylpyrrolidon, Polyvinylacetat, Polycrotonsäure, Polyvinylpropionat, Polymethylvinylether, Polymaleinsäureanhydrid und Polydimethylaminoethylacrylat besteht oder diese enthält.

Der Erfindung liegt die Idee zu Grunde, eine nur temporär bestehende Schutzschicht auf die wirkstoffbeladene Beschichtung aufzutragen und somit diese aktive Beschichtung gegen mechanische Beanspruchung zu schützen. Mit anderen Worten, durch die Auftragung einer Schutzschicht wird die wirkstoffhaltige Beschichtung temporär fixiert. Durch diese Fixierung gelangt der Großteil des Medikamentes zum Bestimmungsort. Die chemische Natur der verwendeten Polymere ermöglicht es dennoch, dass am Bestimmungsort der Wirkstoff quantitativ abgegeben werden kann. Die verwendeten Polymere sind wasserlöslich, gesteuert durch die Molekularmasse geschieht der Auflösungsprozess jedoch nicht spontan, sondern verzögert mit einer Quellungsphase die zwischen 1-10 Min. umfassen kann. Während dieser Zeit ist das Medikament durch den Polymerfilm auf dem Ballonkatheter geschützt. Nach Dilatation wird der vollständig gequollene Film zusammen mit dem Wirkstoff an die Gefäßwand gedrückt und kann dann nach Transfektion seine Wirkung entfalten. Insgesamt ist damit eine homogenere Verteilung des Wirkstoffes an der Läsion gegeben, der Verlust an Wirkstoff auf dem Weg zur Läsion ist reduziert, was zu einer geringeren systemischen Belastung des Patienten führt. Die Schutzschicht bildet demnach eine geschlossene Hülle um die Medikamentenbeschichtung.

Unter einer "pharmazeutisch aktiven Substanz" (oder therapeutisch aktive oder wirksame Substanz) im Sinne der Erfindung wird ein pflanzlicher, tierischer oder synthetischer Wirkstoff (Medikament) verstanden, der in geeigneter Dosierung als Therapeutikum zur Beeinflussung von Zuständen oder Funktionen des Körpers, als Ersatz für natürlich vom menschlichen oder tierischen Körper erzeugte Wirkstoffe sowie zur Beseitigung oder zum Unschädlichmachen von Krankheitserregern, Tumoren, Krebszellen oder Körperfremdstoffen Einsatz findet.

Derartige pharmazeutisch aktive Substanzen weisen beispielsweise eine antiinflammatorische und/oder antiproliferative und/oder spasmolytische Wirkung auf, wodurch beispielsweise Restenosen, Entzündungen oder (Gefäß-)Spasmen vermieden werden können. Derartige Substanzen können insbesondere aus einer oder mehreren Substanzen der Wirkstoffgruppe der Calciumkanalblocker, der Lipidregulatoren (wie beispielsweise Fibrate), der Immunsuppressiva, der Calcineurininhibitoren (wie beispielsweise Tacrolimus), der Antiflogistika (wie beispielsweise Cortison oder Dichlofenac), der Antiinflammatorica (wie beispielsweise Imidazole), der Antiallergika, der Oligonucleotide (wie beispielsweise dODN), der Östrogene (wie beispielsweise Genistein), der Endothelbildner (wie beispielsweise Fibrin), der Steroide, der Proteine, der Hormone, der Insuline, der Zytostatika, der Peptide, der Vasodilatatoren (wie beispielsweise Sartane) und der antiproliferativ wirkenden Stoffe, der Taxole oder Taxane, hier vorzugsweise Paclitaxel, bestehen.

Die pharmazeutische aktive Substanz liegt in der Beschichtung entweder in Reinform oder zusammen mit weiteren pharmazeutischen Hilfsstoffen vor. Als Hilfsstoffe eignen sich beispielsweise Benzalkoniumchlorid, α-Tocopherol, Glucose, Lactose, Calciumphosphat, Calciumhydrogenphosphat, Natriumhydrogencarbonat, Natriumcarbonat, Titanoxid, Zinkoxid, Magnesiumoxid, Silikate wie hochdisperses Siliciumdioxid (Kolloidale Kieselsäure, Aerosil, Si02), Talkum, Kaolin, Bentonit, aliphatische Alkohole, DMSO, Glycerol, Propylenglykol, Stearinsäure, Zucker und Zuckeralkohole, Lactose, Cyclodextrine wie α-Cyclodextrin, β-Cyclodextrin, γ-Cyclodextrin, Mannitol, Sorbitol, Stärken, Cellulosepulver, Celluloseester und -ether wie Methylcellulose, Ethylcellulose, Hydroxyethylcellulose, Hydroxypropylcellulose, Hydroxypropylmethylcellulose, Natriumcarboxymethylcellulose, Celluloseacetatphthalat, Hydroxypropylmethylcelluloseacetatphthalat und mikrokristalline Cellulose, Gelatine, Squalen und andere Isopren-Einheiten, Gummi arabicum, Pektin, Xanthan, Alginate, Polyacrylsäuren wie Carbomere, Polyvinylpyridin, Polyvinylpyrrolidon, Polyvinylalkohol, Polyvinylacetat und Mischungen aus Polyvinylpyridin und Polyvinylacetat, Polyethylenglykol, Vaselin, synthetische und natürliche Fette und Silikone, amphiphile oder oberflächenaktive Hilfsstoffe, wie anionenaktive Tenside, Saponide; kationische Tenside wie Cetylpyridiniumchlorid, nichtionogene Tenside; Polyoxyethylensorbitan, Macrogolglycerolfettsäureester, Fettalkoholether des Polyoxyethylens, Fettsäureester der Saccharose und insbesondere D-α-Tocopheryl-1000-succinat, amphotere Tenside, Komplexemulgatoren wie Cetylstearylalkohol (Typ A und B), quartäre Ammoniumverbindungen und Konservierungsmittel und Antioxidantien wie Citronensäure, Citraconsäure, Weinsäure, Mono- und Polyphosphate, organische Phosphate, wie Dodecylphosphat, Hexosephosphat und Hyaluronidasen oder Hyaluronatlyasen, Butyryl-n-trihexyl-citrat (BTHC) und Triethylcitrat.

Ein weiterer Aspekt der Erfindung liegt in der Verwendung eines entsprechend den vorangehenden Ausführungen ausgestalteten Stents oder Ballonkatheters für die Behandlung koronarer und peripherer Gefäßerkrankungen am Menschen.

### Detaillierte Beschreibung der Erfindung

Herstellung eines mit Paclitaxel beschichteten Stents oder Ballonkatheters über ein zweistufiges Verfahren:
1. Beschichtung mit dem Wirkstoff
   a. Mit Paclitaxel als reinem Wirkstoff oder
   b. Mit Paclitaxel in einer Formulierung zusammen mit einem pharmakologischen Hilfsstoff.
2. Nachbehandlung mit einem filmbildenden Polymer zur kurzfristigen Fixierung der Wirkstoffschicht mit einer Schutzschicht.

Die Schutzschicht bildet demnach eine geschlossene Hülle um die Medikamentenbeschichtung und kann durch geeignete Verfahren aufgebracht werden. Hierzu zählen insbesondere Aufbringen durch Sprühen, Tropfen, Tauchen, Kondensieren, Zerstäuben oder Bedampfen.

Geeignete Hilfsstoffe können beispielsweise aus obig genannter Aufzählung ausgewählt werden.

Die Auftragung der Schutzschicht aus den weiteren genannten Materialien kann in analoger Weise erfolgen.

## Patentansprüche

1. Implantat mit einer wenigstens eine pharmazeutisch aktive Substanz enthaltenden Beschichtung, **dadurch gekennzeichnet, dass** die Beschichtung von einer Schutzschicht bedeckt ist, die aus einem oder mehreren Materialien der Gruppe umfassend Vinylacetat-Crotonsäure-Copolymer, Vinylacetat-Vinylpropionat-Crotonsäure-Terpolymer, Methylvinylether-Maleinsäureanhydrid-Copolymer, Vinylpyrrolidon-Dimethylaminoethylacrylat-Copolymer, Polyvinylpyrrolidon, Polyvinylacetat, Polycrotonsäure, Polyvinylpropionat, Polymethylvinylether, Polymaleinsäureanhydrid und Polydimethylaminoethylacrylat besteht oder diese enthält.

2. Implantat nach Anspruch 1, bei dem die pharmazeutisch aktive Substanz Paclitaxel ist.

3. Implantat nach einem der vorhergehenden Ansprüche, wobei das Implantat ein Stent oder ein Ballonkatheter ist.

4. Implantat nach einem der Ansprüche 1 bis 3 zur Verwendung für die Behandlung koronarer und peripherer Gefäßerkrankungen am Menschen.

## Claims

1. An implant with a coating containing at least one pharmaceutically active substance, **characterised in that** the coating is covered by a protective layer which consists of, or contains one or more materials of the group comprising vinyl acetate-crotonic acid copolymer, vinyl acetate-vinyl propionate-crotonic acid terpolymer, methyl vinyl ether-maleic acid anhydride copolymer, vinylpyrrolidone-dimethylamino ethyl acrylate copolymer, polyvinylpyrrolidone, polyvinyl acetate, polycrotonic acid, polyvinyl propionate, polymethyl vinyl ether, polymaleic acid anhydride, and poly(dimethylaminoethyl acrylate).

2. The implant according to claim 1, in which the pharmaceutically active substance is Paclitaxel.

3. The implant according to either one of the preceding claims, wherein the implant is a stent or a balloon catheter.

4. The implant according to any one of claims 1 to 3 for use for the treatment of coronary and peripheral vascular diseases in humans.

## Revendications

1. Implant doté d'un revêtement contenant au moins une substance pharmaceutiquement active, **caractérisé en ce que** le revêtement est recouvert d'une couche de protection qui est constitué d'un ou de plusieurs matériaux du groupe comprenant un copolymère acétate de vinyle / acide crotonique, un terpolymère acétate de vinyle / propionate de vinyle / acide crotonique, un copolymère méthylvinyléther / anhydride d'acide maléique, un copolymère vinylpyrrolidone / diméthylaminoéthyl acrylate, la poly vinylpyrrolidone, l'acétate de polyvinyle, l'acide polycrotonique, le propionate de polyvinyle, le polyméthylvinyl éther, l'anhydride de l'acide polymaléique et le poly diméthylaminoéthyl acrylate, ou qui contient ceux-ci.

2. Implant selon la revendication 1, chez lequel la substance pharmaceutiquement active est du paclitaxel.

3. Implant selon l'une des revendications précédentes, où l'implant est un stent ou un cathéter à ballon.

4. Implant selon l'une des revendications 1 à 3, pour un emploi pour le traitement de maladies des vaisseaux coronaires et périphériques chez l'humain.
